# EUROPEAN PATENT APPLICATION

(11) **EP 0 803 201 A2**
(43) Date of publication of application: **29.10.1997**
(21) Application number: 97106347.4
(22) Date of filing: 17.04.1997
(51) Int. Cl.: A23L 1/30, A61K 35/78, A23L 1/172

(54) **Dietetic foods and pharmaceutical preparations inhibiting blood coagulation**

(30) Priority: 23.04.1996 EP 96106344
(71) Applicant: CPC INTERNATIONAL INC., Englewood Cliffs New Jersey 07632-9976 (US); CPC Deutschland GmbH, 74074 Heilbronn (DE)
(72) Inventor: Vermeer, Cees, Dr., Maastricht (NL); Gnauck, Günter, Dr., 74248 Ellhofen (DE); Stute, Rolf, Dr., 71686 Remseck (Neckarems) (DE)
(74) Representative: Lederer, Franz, Dr.

(57) **Abstract**

Use of cereal germ oil, preferably corn oil, for the preparation of dietetic foods and pharmaceutical preparations having an inhibiting effect on the blood coagulation. Dietetic foods and pharmaceutical preparations for oral application with a blood anticoagulant effect. A method for inhibiting blood coagulation in humans and warm blooded animals comprising administration by the oral route of cereal germ oil, preferably corn oil.

## Description

### FIELD OF THE INVENTION

The present invention relates to dietetic foods and pharmaceutical preparations for oral applications having a blood anticoagulant effect, which means they are useful for inhibiting blood coagulation in humans and warm blooded animals. The present invention also relates to methods of preparing such dietetic foods and pharmaceutical preparations and to methods of inhibiting blood coagulation.

### BACKGROUND OF THE INVENTION

An increased blood coagulation rate is considered as a risk factor in the development of thrombosis, which can lead to life-threatening complications like heart attack and cardiovascular heart diseases (CHD). Foods having a blood anticoagulant activity could be beneficial in the prevention of CHD.

The process of blood coagulation is a complex mechanism involving many factors including vitamin K-dependent enzymes. Vitamin K belongs to a class of compounds commonly known as prenylquinones. Other prenylquinones, which are found in various foods, include the ubiquinones, in particular ubiquinone-9 and ubiquinone-10. Ubiquinones describe a group of fat-soluble 2,3-dimethoxy-5-methyl-1,4-benzoquinones having an isoprenoid side chain with 1-10 dihydroisoprene-units. The number of the isoprene-residues is indicated by the number attached to the name ubiquinone. The ubiquinones are not considered as vitamins, but act as coenzymes.

In recent years it has been widely recognized that ubiquinone (coenzyme Q,UQ), in addition to its involvement as an electron and proton carrier in mitochondrial and bacterial respiration, acts in its reduced form (ubiquinol) as an antioxidant. An an antioxidant, it can protect unsaturated fatty acids against lipid peroxidation and also help to minimize the damages caused by the degenerative diseases of aging, such as brain dysfunction, cataracts, CHD and cancer. This is especially true for UQ 10, which has been demonstrated in various clinical and in vitro studies. Here, special pharmaceuticals are available. Bracco, et al., European Patent No. 424 679 discloses the protection of a fat or a food, cosmetic or pharmaceutical product containing a fat, against oxidation by incorporation of coenzyme Q, in particular coenzyme Q₁₀.

Recently, it was found that synthetic derivatives of prenylquinones inhibit the vitamin K-dependent enzyme Υ-glutamylcarboxylase and, to a minor extent, the vitamin K-epoxide reductase. The present invention is based on the finding that ubiquinones may also affect the vitamin K-dependent enzymes in vitro. They were found to inhibit both, the vitamin K-dependent carboxylase and the K-epoxide reductase in a rat as well as in a cow liver system; 50% inhibition was obtained at concentrations in the micromolar range. It has been, therefore, concluded that ubiquinones (besides their beneficial effect on e.g. lipid peroxidation, heart function, blood pressure and arteriosclerosis), may be potential antagonists of vitamin K and may interfere with vitamin K-dependent reactions in vivo, which play an important role in blood coagulation.

In-vivo-experiments demonstrate, however, that ubiquinones, when administered orally, do not have such an effect. When administered intravenously (about 6mg/kg bw.), ubiquinones have an inhibiting effect on blood coagulation parameters. Surprisingly, however, it was found that cereal germ oils, especially corn (maize) germ oil, which contain ubiquinones, had a distinct anticoagulant effect when fed to test animals (rats) in a diet containing 45% energy as corn oil.

When extrapolating the animal data to humans (adult reference person of about 60 kg) the effective dose would be equivalent to about 40 mg Ubiquinone 9 or 100 g corn oil. In the case of oil enriched with ubiquinone, the effective dose of corn oil can be substantially reduced to about 10-20 ml.

It is not sure, whether the anticoagulant effect of germ oils is due to their content of ubiquinones or to other ingredients in corn oil, which act synergistically. As a matter of fact, cereal germ oils, in particular corn oil, had a pronounced inhibiting effect on the blood coagulation, when administered orally, while other oils like soy bean, coconut or sunflower oil did not show this effect or, if it all, to a minor extent.

The effect might be due to a solubilizing action of the germ oil or to a synergistic action of other ingredients in the oil.

### SUMMARY OF THE INVENTION

The invention, therefore, relates to the use of cereal germ oil, preferably corn oil, for the preparation of dietetic foods and pharmaceutical preparations having an inhibiting effect on blood coagulation, to dietetic foods and pharmaceutical preparations for oral application with a blood anticoagulant effect and also to a method for inhibiting blood coagulation in humans and warm blooded animals comprising administration by the oral route of cereal germ oil, preferably corn oil.

### DETAILED DESCRIPTION OF THE INVENTION

The present invention relates to the use of cereal germ oil, preferably corn (maize) germ oil, for the preparation of dietetic foods and pharmaceutical preparations having an inhibiting effect on the blood coagulation.

In addition to the beneficial effect of unsaturated fatty acids on the cholesterol metabolism and on blood pressure regulation, according to the present invention, corn oil, surprisingly, upon oral administration, has a distinguished beneficial effect to protect against thrombosis and related diseases.

In-vivo-experiments have shown that the intake of corn germ oil induces
a) a decrease in plasma prothrombin, and
b) a parallel increase in the hepatic prothrombin precursor molecules.

Further data have been generated in an "in-vivo-experimental animal-model" for arterial thrombosis tendency. This model is generally known as the "aorta loop model".

The aorta loop model is an experimental setup to measure the effect of nutrients on the occlusive arterial thrombosis in rats (Hornstra et al 1975 Atherosclerosis 22, 499-516). The rats are subjected to a given treatment, and after a certain time-period the abdominal aorta is cut across 3 mm below the spermatic arteries, to allow the insertion of a loop-shaped polyethylene cannula. The wound is connected in such a way that the loop protrudes from the abdomen and projects to the external environment.

At places where the aorta-loop is in permanent contact with the vessel wall, endothelial damage and flow disturbances result in the production and growth of a thrombus, which reaches an occlusive state about 5 days after surgery. This moment of total obstruction is indicated by a change of color of the blood in the translucent loop. The period between insertion and complete obstruction of the loop is called the obstruction time (OT), and may be used as a measure for the arterial thrombosis tendency in the animal: The shorter the OT is, the higher is the arterial thrombosis tendency. The model has been widely used for demonstrating the effects of saturated and unsaturated fatty acids on the thrombosis tendency. Normally hardened coconut oil is taken as a positive control in this test.

Use of cereal germ oil according to the present invention in particular on regular consumption may help to prevent thromboembolic and cardiovascular diseases. Presently, anticoagulants of the coumarin type are frequently used for that purpose: normal dose treatment to prevent thrombosis and myocardial infarction and low dose treatment to prevent peripheral arterial diseases.

Corn oil might be a natural anticoagulant, the effect of which is comparable with the low dose coumarin treatment. The main difference is that the effect of corn oil on the recycling of vitamin K is reversible, that of coumarins not. Therefore, patients on coumarin anticoagulants need to be controlled frequently (risk of bleeding), whereas products containing corn germ-oil can be used freely.

The dietetic foods and pharmaceutical preparations according to the invention contain this cereal germ oil, preferably corn (maize) germ oil, and an appropriate basic foodstuff or suitable carrier, respectively.

The following examples demonstrate the anticoagulant activity of corn oil, as well as prenylquinones present in corn oil, and are not meant to be limiting in any way.

### Example 1

### Effect of dietary oils on plasma prothrombin concentration (rat feeding study)

Wistar rats were fed during 12 weeks ad libitum with a diet containing 45 energy % of one of the oils mentioned in Table 1. Each group of rats consisted of 20 animals. Blood samples were taken at the end of the test by venipuncture of the rat's tail vein. As a blood coagulation marker, the prothrombin concentrations were determined with a coagulometer (KC-4, Amelung, Germany) using a commercial thromboplastin preparation (Thromborel S®) and clotting factor II-deficient plasma (both from Behringwerke AG, Marburg, Germany). Prothrombin concentrations were calculated with the aid of a reference curve from pooled normal rat plasma. Prothrombin is an important factor in the process of blood coagulation. The antithrombotic properties directly correlate to the decrease in plasma prothrombin.

The consumption of corn oil by the rats resulted in a significant prolongation of their blood coagulation time, caused by a decrease in the "vitamin K-dependent" coagulation factor prothrombin. No such prolongation was found for the other oils, such as coconut, soybean and sunflower oil. The superior anti-thrombotic effect is clearly demonstrated in Table 1. Each number listed in Table 1 represents the average of 20 prothrombin measurements ± SD.

**Table 1**

| **Effect of dietary oils on plasma prothrombin concentrations** | |
|---|---|
| Type of oil | Prothrombin concentration (% of control group) |
| soybean oil | 84 ± 9 |
| coconut oil | 83 ± 9 |
| sunflower oil | 87 ± 10 |
| corn germ oil | 51 ± 8 |

### Example 2

### Effect of ubiquinone/plastoquinone on vitamin K dependent enzymes in vitro

Prenylquinones like phylloquinone (vitamin K₁), ubiquinone and plastoquinone function as co-enzymes during the conversion of glutamate into γ-carboxyl-glutamate (Gla), which is an essential step in the formation of the blood coagulation factors. The active form of vitamin K is the quinol (KH₂), the oxidation of which provides the energy required for Gla formation. In this reaction KH₂ is converted into vitamin K epoxide (KO), which is recycled in two steps by the action of an enzyme called KO-reductase.

In vitro systems were used to test either the γ-glutamylcarboxylase or KO-reductase. Salt-washed microsomes were prepared from the livers of normal cows and from normal and vitamin K-deficient rats of the Lewis strain.

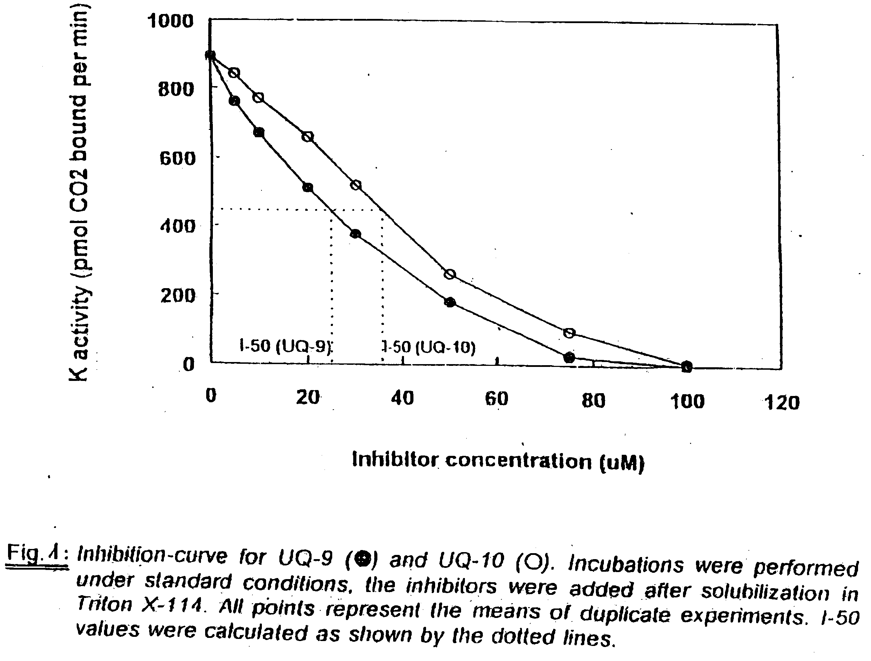

Inhibition curves were prepared at a wide concentration range of prenylquinones (UQ-9, UQ-10, and PQ-9) using the cofactor vitamin K either in its quinol, its quinone, or in its epoxide form. An example of such an inhibition curve is shown in Fig. 1. From these curves the inhibitor concentrations required for 50% inhibition (I-50) were calculated (dotted lines in Fig. 1) for the various carboxylating enzyme systems from the cow as well as from rats. Incubations were performed under standard conditions, the inhibitors were added after solubilization in Triton X-114. All points in Fig. 1 represent the means of duplicate experiments. I-50 values were calculated as shown by the dotted lines.

The data obtained for all inhibitors are summarized in Table 2, and demonstrate that all I-50 values range between 30 and 150 µM.

**Table 2**

| **Effect of prenylquinones on Υ-glutamylcarboxylase activity** | | | | | | |
|---|---|---|---|---|---|---|
| | I-50 (µm) in bovine system | | | I-50 (µM) in rat system | | |
| Cofactor: | KH2 | K | KO | KH2 | K | KO |
| Inhibitor: | | | | | | |
| UQ-10 | 110 ± 17 | 76 ± 5 | 91 ± 15 | 36 ± 7 | n.d. | n.d. |
| UQ-9 | 105 ± 14 | 86 ± 12 | 72 ± 8 | 25 ± 6 | n.d. | n.d. |
| PQ-9 | 85 ± 11 | 59 ± 4 | 41± 7 | 39 ± 10 | n.d. | n.d. |
| *All data are expressed as the means of triplicate experiments with S.E.M.* *"n.d." stands for not determined because of instability of the enzyme.* | | | | | | |

All natural prenyl-benzoquinones tested had inhibitory activity for both Υ-glutamylcarboxylase and KO-reductase; both enzymes were inhibited to a comparable extent. This demonstrates the potency of ubiquinone/plastoquinone with regard to anticoagulant activity.

### Example 3

Example 3 demonstrates the high antithrombotic activity of corn oil.

### Aorta loop model:

Male Wistar rats entered the experiment at the age of 5 weeks, and were fed ad libitum with diets containing 45 energy % of either hardened coconut oil, sunflower seed oil, or corn oil for a period of 8 weeks. Three groups consisting of 42 rats each were compared. After 8 weeks the aorta loops were implanted, and the diets were continued until the obstruction took place. The time required before the loop was obstructed by a sold thrombus (the obstruction time) was taken as a measure for thrombosis tendency. The longer the obstruction time the lower is the thrombosis tendency. The precise registration of the obstruction times required regular day and night inspection of the animals.

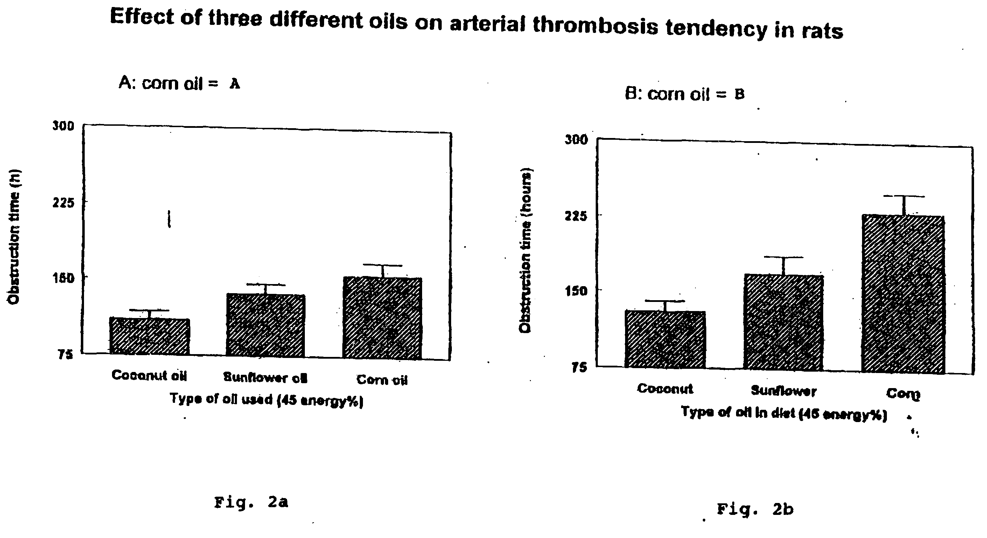

Two experiments A and B were conducted. In experiment A, a corn oil from the Dutch market, with unknown history, was used. In experiment B, a corn oil of known origin and processing condition, characterized by a higher ubiquinone content (see Table 3), was used. The effect of three different oils on arterial thrombosis tendency in rats is shown in Figures 2a and 2b.

The mean obstruction times in experiment A for the coconut oil, the sunflower seed oil, and the corn oil group were 91, 123 and 144 hours respectively (Figure 2a). There was a substantial (35%) increase in obstruction time between hardened coconut oil and sunflower seed oil, which is consistent with earlier observations. It is also clear that the mean obstruction times for the corn oil group were still further prolonged (58% relative to coconut oil, which is almost twice as much as with sunflower oil).

In experiment B, using the corn oil with the higher concentration of ubiquinone, these differences in favor of corn oil were even more pronounced (Figure 2 b). This result is well in line with the ubiquinone derived effects on the vitamin K dependent enzymes in vitro as described in Example 2.

**Table 3**

| **Ubiquinone and Tocopherol contents (ppm) of different corn oils used in the aorta loop experiments A and B** | | |
|---|---|---|
| | Corn oil Exp. A | Corn oil Exp. B |
| Ubiquinone Q 9 | 264 | 306 |
| Ubiquinone Q 10 | 7 | 8 |
| α-Tocopherol | 115 | 159 |
| α-Tocotrienol | 5 | 7 |
| y-Tocopherol | 892 | 953 |
| y-Tocotrienol | 31 | 31 |

### Example 4

### Effect of Mazola® corn oil intake on antithrombotic parameters in human beings

Beneficial effects on antithrombotic blood parameters have also been obtained in a human pilot study. During a period of 14 days, four test persons received 80 ml of Mazola® corn oil per day. The following blood parameters have been measured at day 0 and day 14:
Prothrombin, coagulation factor VII, triglycerides and vitamin K.
A remarkable reduction of the starting values correlates well with the antithrombotic effects observed in the former examples (Table 4).

**Table 4**

| **Changes of selected blood parameters after a daily intake of 80 ml Mazola® corn oil** | | |
|---|---|---|
| | Day 0 | Day 14 |
| Prothrombin* (%) | 114 | 104 |
| Coagulation factor VII* (%) | 105 | 101 |
| Triglycerides (mmol/l) | 0.88 | 0.68 |
| Vitamin K (ng/ml) | 0.71 | 0.53 |

| | | |
|---|---|---|
| *data are given as a percentage of a pooled normal plasma reference level | | |

The invention now being fully described, it will be apparent to one of ordinary skill in the art that many changes and modifications can be made thereto without departing from the spirit or scope of the invention as set forth herein.

## Claims

1. Use of cereal gem oil, preferably corn (maize) germ oil, for the preparation of dietetic foods and pharmaceutical preparations having an inhibiting effect on the blood coagulation.

2. Dietetic foods and pharmaceutical preparations for oral application with a blood anticoagulant effect comprising cereal germ oil, preferably corn (maize) germ oil, in a suitable carrier.

3. Dietetic food and pharmaceutical preparations according to claim 2 wherein a germ oil is used which has been enriched in ubiquinone 9/10.

4. Dietetic foods and pharmaceutical preparations according to claim 3 wherein a germ oil is used which by CO₂-extraction resp. fractionation has been enriched in ubiquinone 9/10.

5. Method for inhibiting blood coagulation in humans and warm blooded animals comprising administration by the oral route of cereal germ oil, preferably corn (maize) germ oil, having a natural or enriched concentration of ubiquinone 9/10.
